# EUROPÄISCHE PATENTSCHRIFT

(11) **EP 0 604 675 B1**
(45) Veröffentlichungstag und Bekanntmachung des Hinweises auf die Patenterteilung: **08.05.1996**
(21) Anmeldenummer: 92122024.0
(22) Anmeldetag: 28.12.1992
(51) Int. Cl.: A61F 6/04, A61B 19/04, B65D 85/00

(54) **Verpackungseinheit**
Package unit
Unité d'emballage

(43) Veröffentlichungstag der Anmeldung: 06.07.1994
(73) Patentinhaber: Maxeiner, Hank, D-56068 Koblenz (DE)
(72) Erfinder: Maxeiner, Hank, D-56068 Koblenz (DE)
(74) Vertreter: Hentschel, Peter, Dipl.-Ing.

(56) Entgegenhaltungen:
- DE-U- 9 213 053
- US-A- 4 875 491
- US-A- 4 987 905
- US-A- 5 065 863

## Beschreibung

Die Erfindung betrifft eine Verpackungseinheit, bestehend aus einer Umverpackung und einem darin angeordnetem Kondom in Form einer hochelastischen, schlauchförmigen, einendig geschlossenen Hülle, die in Verpackungslage um einen ringförmigen Randwulst am offenen Ende der Hülle aufgerollt ist, und in die streifenförmige Glieder aus dünnem, flexiblem und weichem Material als Abrollhilfen über den größten Teil ihrer Länge miteingerollt sind, wobei die Umverpackung aus reißfähigem Material besteht und in den an der bzw. gegenüber der Kondomöffnung liegenden Wandungen Sollbruchstellen z.B. Solleinreißlinien aufweist und wobei die über das eingerollte Kondom vorstehenden Enden der über den Umfang des Kondoms gleichmäßig verteilt angeordneten, streifenförmigen, mit ihren anderen Enden in das Kondom eingerollten Glieder im wesentlichen koaxial um das Kondom innen an der Umverpackung fixiert sind. Eine solche Verpackungseinheit ist beispielsweise aus der US-A-4987905 bekannt.

Mit Hilfe der bekannten Verpackungseinheit ist es möglich, Kondome oder Präservative, schneller, sicherer und schonender benutzbar zu machen.

Sie ermöglicht ein Überziehen des Kondoms,ohne daß ein vorheriges Aufreißen der Verpackung und Herausnehmen des Kondoms erforderlich sind. Beim mechanischen Druck von der Unterseite der Verpackung her reißen die Sollbruchstellen und der Penis dringt in das Kondom ein. Beim Abrollen des Kondoms werden die im wesentlichen koaxial und sternförmig um den Kondom an der Packung innen befestigten, eingerollten, streifenförmigen Glieder mit abgerollt. Diese wirken einerseits in vorteilhafter Weise als Abrollhilfe, andererseits sichern diese darüber hinaus eine gleichmäßige Druckverteilung auf das Material des Kondoms beim Anlegen desselben, so daß keine Spannungsspitzen auftreten und auch eine mechanische Beschädigung des empfindlichen Kondommaterials verhindert wird.

Ausgehend von diesem Stand der Technik liegt der Erfindung die Aufgabe zugrunde, eine Verpackungseinheit der eingangs genannten Art zu schaffen, die unter Beibehaltung der Vorteile der bekannten Verpackungseinheit eine vereinfachte, maschinelle Produktion ermöglicht. Darüber hinaus soll sie ein einfaches, seitlichen Abziehen der Reste der Umverpackung mitsamt den abgerollten, streifenförmigen Gliedern nach Anlegen des Kondoms ermöglichen.

Diese Aufgabe wird erfindungsgemäß durch zwei Alternativen gelöst. Die erste Alternative ist dadurch gekennzeichnet, daß die nicht eingerollten Enden der streifenförmigen Glieder miteinander verbunden und in das Material der Umverpackung miteingeformt sind und daß ferner mindestens eine quer zum eingerollten Kondom verlaufende Sollbruchstelle z.B Solleinreißlinie im Bereich der zusammenhängenden Enden der streifenförmigen Glieder vorgesehen ist.

Dadurch, daß die nicht eingerollten Enden der streifenförmigen Glieder miteinander verbunden und in das Material der Umverpackung miteingeformt sind, ist eine vereinfachte, maschinelle Produktion möglich. Die Anordnung einer Sollbruchstelle im Bereich der zusammenhängenden Enden der streifenförmigen Glieder ermöglicht ein einfaches, seitliches Abziehen der Reste der Umverpackung mitsamt den abgerollten, streifenförmigen Gliedern nach Anlegen des Kondoms.

Die zweite Alternative ist dadurch gekennzeichnet, daß die nicht eingerollten Enden der streifenförmigen Glieder an einem eine querverlaufende zum Abziehen der Umverpackung (4) vorgesehene Unterbrechung aufweisenden Ring aus weichem, flexiblen Material angeformt sind, der in das Material der Umverpackung (4) miteingeformt ist und im wesentlichen koaxial zum eingerollten Kondom angeordnet ist.

Auch wenn die nicht eingerollten Enden der streifenförmigen Glieder in vorteilhafter Weise an einem offenen Kunststoffring angeformt sind, so bildet auch diese Anordnung eine weiche, variable Öffnung, so daß sich die gesamte Verpackungseinheit bei Druck leicht über den eindringenden Penis stülpen und nach Überstreifen mitsamt den Resten der Umverpackung von diesem abgezogen und weggeworfen werden kann. Darüber hinaus ermöglicht diese Ausgestaltungsform in vorteilhafter Weise das Anlegen des Kondoms mit nur einer Hand, so daß auch Einarmige dieses Kondom ohne fremde Hilfe benutzen können.

Gemäß Anspruch 2 ist in vorteilhafter Weiterbildung der Erfindung vorgesehen, daß die streifenförmigen Glieder so angeordnet und geformt sind, daß sie sich im oberen Bereich einander seitlich überlappen.

Hierdurch wird erreicht, daß das empfindliche Material des Kondoms von den Fingernägeln der Hand beim Anlegen nicht beschädigt wird. Darüber hinaus ist - wie bereits oben ausgeführt - eine gleichmäßige Druckverteilung auf das Kondom beim Anlegen gewährleistet.

Gemäß Anspruch 3 ist in vorteilhafter Weise vorgesehen, daß die Umverpackung einen rechteckigen oder quadratischen Umriß aufweist und die Sollbruchstellen z.B. Solleinrißlinien in den Wandungen der Umverpackung als Diagonalkreuz angeordnet sind. Genausogut kann die Umverpackung aber auch einen runden oder ovalen Umriß aufweisen.

Die Sollbruchstellen z.B. Solleinrißlinien können aber gemäß Anspruch 4 in vorteilhafter Weise auch kreisförmig angeordnet sein.

Auch ist es möglich, die Sollbruchstellen z.B. Solleinrißlinien gemäß Anspruch 5 in den Wandungen der Umverpackung entlang der Mittellinien derselben anzuordnen. Zusätzlich kann noch im Mündungsbereich dieser Mittellinien - Sollbruchstellen z.B Solleinrißlinien in die Seitenkanten der Umverpackung Einreißhilfen in Form von Einkerbungen, Einschnitten oder Ausnehmungen o.dgl. angeordnet sein.

Alle drei Ausgestaltungen ermöglichen in vorteilhafter Weise in Verbindung mit der Reißfähigkeit des Verpackungsmaterials ein leichtes und sicheres Aufreißen der erfindungsgemäßen Verpackungseinheit bei relativ geringem, mechanischen Druck.

In weiterer vorteilhafter Ausgestaltung der Erfindung können sich gemäß Anspruch 6 die streifenförmigen Glieder zu ihren freien Enden hin verjüngen oder gemäß Anspruch 7 auch abgerundet sein. Diese Ausgestaltungen kommen in vorteilhafter Weise der Handhabung sowie der Materialersparnis zugute.

Vorteilhaft ist weiterhin, daß gemäß Anspruch 8 die streifenförmigen Glieder breite und dünne Bänder aus Kunststoff sind und gemäß Anspruch 9 als Kunststoff Polypropylen oder Polyäthylen verwendet ist.

Mit Hilfe der erfindungsgemäßen Verpackungseinheit wird ein Einhand-Schnellüberzieh-System geschaffen.

Das Prinzip beruht darauf, das hochflexible und empfindliche Gummimaterial in sehr dünne, weiche flexible aber feste Kunststoffstreifen einzurollen. Diese Kunststoffstreifen werden in einem Stück mit der Umverpackung hergestellt, so daß Umverpackung und die Abrollvorrichtung in Form der Kunststoffstreifen aus einem Stück bestehen. Die Streifen sind etwa 15 cm lang und verjüngen sich nach unten. Um sich das Aufreißen der Packung mit zwei Händen zu ersparen, besitzt die Umverpackung oben und unten Sollbruchstellen. Die Umverpackung selbst wird ringsherum auf gewöhnliche Weise verschweißt oder verklebt.

Die Packung ist so beschaffen, daß bei normaler Handhabung Hygiene und Unversehrtheit des Inhalts gewährleistet werden. Bei mechanischem Druck von der Unterseite reißt die Sollbruchstelle unten. Gleichzeitig werden durch den Druck von Zeige- und Mittelfinger die in das Kondom eingerollten Kunststoffstreifen über den eindringenden Gegenstand (Penis) abgerollt. Die Dicke des eindringenden Gegenstandes ist dabei von untergeordneter Bedeutung, da z.B. das Kondom an sich flexibel ist und die Kunststoffstreifen durch ihre kreisförmige und am oberen Drittel überlappende Anordnung eine variable öffnung ermöglichen und sich bei Druck vom oben über den eindringenden Gegenstand stülpen.

Die Umverpackung ist so beschaffen, daß nach Beendigung des Abrollvorgangs die ganze Umverpackung einschließlich der Plastikstreifen freiliegt und die Sollbruchstellen eingerissen sind.

Die ganze Umverpackung kann nach Beendigung des Abrollvorgangs komplett seitlich eingerissen und abgezogen werden. Damit ist der ganze Überzievorgang mit einer Hand ausgeführt. Für den gesamten, einhändigen Abrollvorgang werden nur etwa 5 Sekunden benötigt.

Die Erfindung ist anhand von Ausführungsbeispielen schematisch in den Zeichnungen dargestellt und näher beschrieben. Es zeigt:
- Fig. 1 -: eine räumliche Ansicht einer erfindungsgemäßen Verpackungseinheit, wobei die Wandung auf der Oberseite durchsichtig dargestellt ist, so daß das eingerollte Kondom und die eingearbeiteten Abrollhilfen sichtbar sind,
- Fig. 2 -: eine schematisierte Teil-Schnittdarstellung durch den aufgerollten Kondom und die eingerolltem Kunststoffstreifen ohne Umverpackung, in stark vergrößerter Darstellung,
- Fig. 3 -: eine räumliche Ansicht der Verpackungseinheit während des Aufsetzens auf den Penis und Druckaufbringung mit zwei Fingern einer Hand kurz vor dem Aufreißen der Verpackungseinheit,
- Fig. 4 -: eine Draufsicht auf eine Wandung der Umverpackung mit an dieselbe angeformte und in die Bildebene ausgebreiteten Kunststoffstreifen ohne Kondom und ohne Deckwandung und
- Fig. 5 -: eine Draufsicht auf eine Wandung der Verpackungseinheit mit darin integriertem Kunststoffring mit daran befestigten und in die Bildebene ausgebreiteten Kunststoffstreifen, ohne Kondom und ohne Deckwandung.

Die in den Zeichnungen schematisch dargestellte Verpackungseinheit 1 enthält einen Kondom 2 in Form einer einendig kalottenförmig geschlossenen, mit einer oberen Ausbeulung als Reservoir 2 a versehenen, etwa zylindrischen Hülle, die in Verpackungslage um einen ringförmigen Randwulst 2 b an ihrem offenen Ende aufgerollt ist und in die streifenförmige Glieder 3 als Abrollhilfen in Form von dünnen flexiblen Bändern über den Umfang verteilt eingerollt sind. Die Enden 3 a der streifenförmigen Glieder 3 bzw. Bänder ragen in Verpackungslage über den aufgerollten Kondom hinaus und sind innen in die Umverpackung 4 integriert.

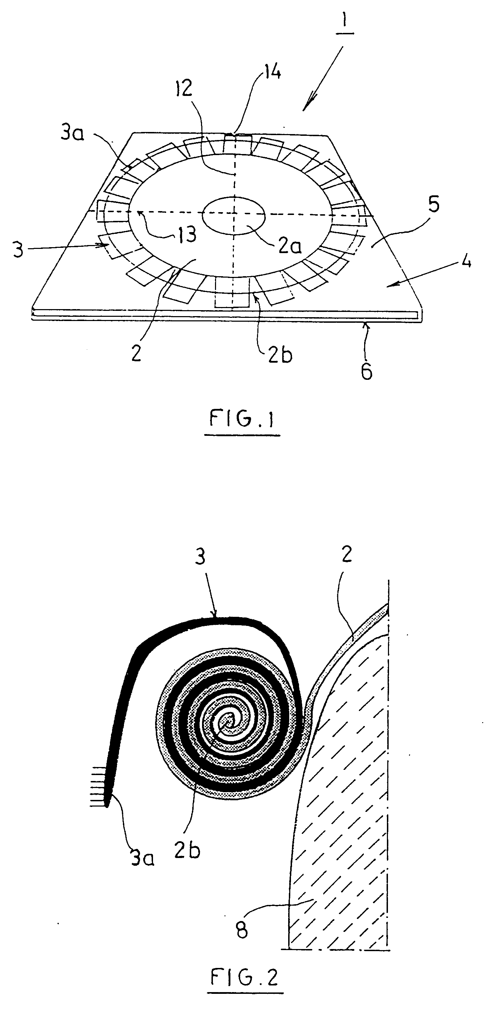

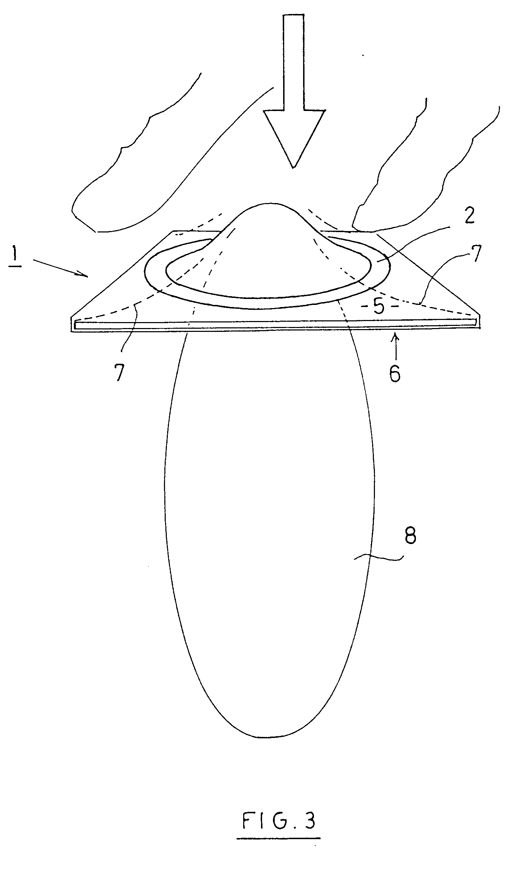

Die Umverpackung 4 besteht aus reißfähigem Material und besitzt in den bzw. an den gegenüber der Kondomöffnung liegenden Wandungen 5, 6, also in der oberen und unteren Wandung Umverpackung 4, Sollbruchstellen , die in den in Fig. 3, 4 und 5 dargestellten Ausführungsbeispielen in Form von Diagonalkreuzen 7 angeordnet sind. Die Sollbruchstellen in den Wandungen 5 und 6 können jedoch auch kreisförmige Gestalt haben. Es können auch Sollbruchstellen 12 und 13 so angeordnet werden, daß sie entlang den Mittellinien verlaufen (Fig. 1) oder I-förmige Gestalt haben.

Zum Anlegen des Kondoms 2 wird auf die Verpackungseinheit nach Aufsetzen mit ihrer unterseitigen Wandung 6 auf den Penis 8 z.B. mittels Zeige- und Mittelfinger ein mechanischer Druck ausgeübt, wodurch zunächst die Sollbruchstellen der unteren Wandung 6 der Umverpackung 4 einreißen, so daß der Penis in das eingerollte Kondom von unten, d.h. von dessen offenem Ende her, automatisch hineinstößt, wodurch gleichzeitig auch auf die Sollbruchstellen in der oberen Wandung 5 Druck aufgebracht wird und diese ebenfalls aufreißt. Gleichzeitig mit dieser Bewegung wird beim Herunterdrücken der Umverpackung 4 der Abrollvorgang in Gang gesetzt, bei dem die eingerollten Bänder 3 für ein schonendes Abrollen des Kondoms 2 sorgen, ohne daß die Hand mit dem empfindlichen Kondommaterial in direkte Berührung kommt.

Um den Abrollvorgang besonders sicher und schonend zu gestalten, ist es vorteilhafter Weise erfindungsgemäß vorgesehen, daß die eingerollten Bänder 3 einander seitlich überlappen. Zur Erhöhung der Bedienungsfreundlichkeit und zur Materialersparnis können die Bänder 3 sich zu ihrem freien Ende hin auch verjüngen und zudem an ihren Enden abgerundet sein.

Vorteilhafterweise ist erfindungsgemäß vorgesehen, daß zur Unterscheidung von Ober- und Unterseite der Verpackungseinheit diese mit einem entsprechenden Aufdruck oder Kennzeichnung versehen ist, damit sichergestellt ist, daß die Verpackungseinheit 1 richtig gehandhabt wird. Auch kann die Verpackung zu diesem Zweck aus durchsichtigem Material hergestellt sein. Es kann auch eine fluorsierende Kennzeichnung verwendet werden, um die sichere Benutzung der Handhabungseinheit auch in der Dunkelheit zu ermöglichen.

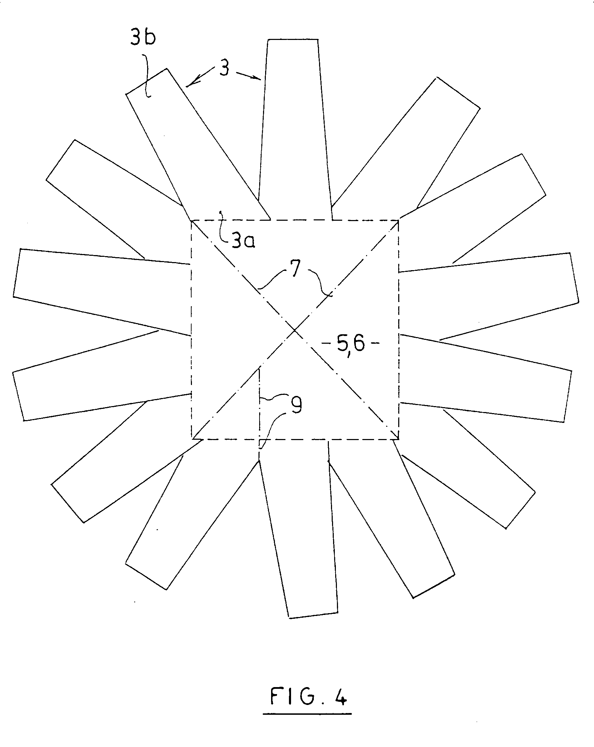

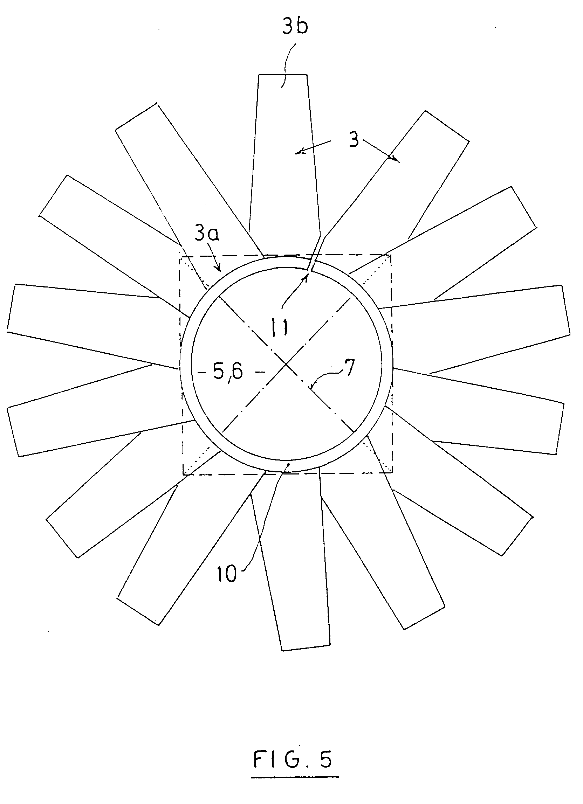

Ist die Abrollbewegung des Kondoms 2 beendet, können die Reste der Verpackungseinheit 1 einschließlich der Abrollstreifen (streifenförmigen Glieder) 3 dank der hierfür vorgesehenen weiteren Sollbruchstellen 9, z.B. bei der Ausführungsform gem. Fig. 4 - seitlich eingerissen oder abgeschert und abgezogen werden. Gemäß einer anderen vorteilhaften Ausführungsform der Erfindung können die streifenförmigen Glieder 3 oder Bänder an einem eine querverlaufende Unterbrechnung (Spalt) 11 aufweisenden Ring 10 aus flexiblem, weichen Material, vorzugsweise Kunststoff, angeformt sein. Dieser Ring 10 mit den daran befestigten Enden 3 a der streifenförmigen Bänder 3 ist im wesentlichen koaxial zum eingerollten Kondom 2 in die untere Wandung 6 der Umverpackung 4 eingeformt (Fig. 5).

Dadurch, daß der Ring 10 mit den streifenförmigen Gliedern 3 (Abrollbänder) durch einen Spalt 11 unterbrochen und flexibel ist, läßt sich dieser zusammen mit den Resten der Umverpackung 4 nach dem Abrollvorgang ebenfalls auf einfache Weise durch seitliches Abziehen entfernen.

Die Erfindung ist nicht nur auf die dargestellten und beschriebenen Ausführungsbeispiele beschränkt. Vielmehr stellen diese nur vorteilhafte Ausgestaltungsformen des Erfindungsgedankens dar.

So kann in vorteilhafter Weise zusätzlich zu einer bspw. kreisförmig geschlossenen Sollbruchsteile auf der Unterseite der Umverpackung auch eine Lasche mit dem aufzureißenden Bereich derart befestigt sein, daß die aufgeklebte Lasche erfaßt und beim Weiterreißen der vorgeprägte Verpackungsbereich mitgerissen wird. Hierdurch wird erreicht, daß dieser Bereich der Verpackung auf der Unterseite derselben schon vor Aufsetzen auf den Penis mit der Hand geöffnet werden kann (Anspruch 14).

Der Schutzumfang der Erfindung erstreckt sich auf die Merkmale der einzelnen Ansprüche.

## Patentansprüche

1. Verpackungseinheit, bestehend aus einer Umverpackung (4) und einem darin angeordnetem Kondom (2) in Form einer hochelastischen, schlauchförmigen, einendig geschlossenen Hülle, die in Verpackungslage um einen ringförmigen Randwulst am offenen Ende der Hülle aufgerollt ist, und in die streifenförmige Glieder (3) aus dünnem, flexiblem und weichem Material als Abrollhilfen über den größten Teil ihrer Länge miteingerollt sind, wobei die Umverpackung (4) aus reißfähigem Material besteht und in den an der bzw. gegenüber der Kondomöffnung liegenden Wandungen (5 und 6) Sollbruchstellen z.B. Solleinreißlinien (7 bzw. 12, 13) aufweist und wobei die über das eingerollte Kondom (2) vorstehenden Enden (3 a) der über den Umfang des Kondoms gleichmäßig verteilt angeordneten, streifenförmigen, mit ihren anderen Enden (3 b) in das Kondom (2) eingerollten Glieder (3) im wesentlichen koaxial um das Kondom (2) innen an der Umverpackung (4) fixiert sind,
dadurch gekennzeichnet,
daß die nicht eingerollten Enden (3 a) der streifenförmigen Glieder (3) miteinander verbunden und in das Material der Umverpackung (4) miteingeformt sind und daß ferner mindestens eine quer zum eingerollten Kondom verlaufende Sollbruchstelle z.B. Solleinreißlinie (9) im Bereich der zusammenhängenden Enden (3 a) der streifenförmigen Glieder (3) zum Abziehen der Umverpackung (4) mit den streifenförmigen Gliedern (3) vorgesehen ist, oder die nicht eingerollten Enden der streifenförmigen Glieder (3) an einem eine querverlaufende zum Abziehen der Umverpackung (4) vorgesehene Unterbrechung (11) aufweisenden Ring (10) aus weichem, flexiblen Material angeformt sind, der in das Material der Umverpackung (4) miteingeformt ist und im wesentlichen koaxial zum eingerollten Kondom (2) angeordnet ist.

2. Verpackungseinheit nach Anspruch 1,
dadurch gekennzeichnet,
daß die streifenförmigen Glieder (3) so angeordnet und geformt sind, daß sie sich im oberen Bereich einander seitlich überlappen.

3. Verpackungseinheit nach Anspruch 1,
dadurch gekennzeichnet,
daß die Umverpackung (4) einen rechteckigen oder quadratischen oder runden oder ovalen Umriß aufweist und die Sollbruchstellen z.B. Solleinreißlinien (7) in den Wandungen (5, 6) der Umverpackung (4) als Diagonalkreuz angeordnet sind.

4. Verpackungseinheit nach Anspruch 1,
dadurch gekennzeichnet,
daß die Sollbruchstellen z.B. Solleinreißlinien in den Wandungen (5, 6) der Umverpackung (4) kreisförmig angeordnet sind.

5. Verpackungseinheit nach Anspruch 1,
dadurch gekennzeichnet,
daß die Sollbruchstellen (7) in den Wandungen (5, 6) der Umverpackung (4) entlang der Mittellinien (12 und/oder 13) zu derselben verlaufen und im Mündungsbereich derselben in den Seitenkanten der Umverpackung (4) Einreißhilfen (14) in Form von Einkerbungen, Einschnitten, Ausnehmungen o.dgl. angeordnet sind.

6. Verpackungseinheit nach Anspruch 1,
dadurch gekennzeichnet,
daß die streifenförmigen Glieder (3) zu ihren freien Enden (3 b) hin sich verjüngen.

7. Verpackungseinheit nach Anspruch 1,
dadurch gekennzeichnet,
daß die streifenförmigen Glieder (3) an ihren freien Enden (3 b) abgerundet sind.

8. Verpackungseinheit nach Anspruch 1,
dadurch gekennzeichnet,
daß die streifenförmigen Glieder (3) breite und dünne Bänder aus Kunststoff sind.

9. Verpackungseinheit nach Anspruch 1,
dadurch gekennzeichnet,
daß als Kunststoff Polypropylen oder Polyäthylen verwendet ist.

10. Verpackungseinheit nach Anspruch 1,
dadurch gekennzeichnet,
daß ein durch Sollbruchstellen vorgeprägter Bereich auf der unteren Wandung (6) der Umverpackung (4) mit einer Abreißlasche herausreißbar angeordnet ist.

## Claims

1. Package unit comprising an outer wrap (4) and a condom (2) therein having the form of a highly elastic tube sealed at one of its ends which in packaged condition is rolled up around an annular marginal bead at the open end thereof and which has strip-like members (3) of a thin flexible and soft material rolled into it over most of its length that are to serve as unrolling aids,
wherein said outer wrap (4) consists of tear-resistant material and is provided with rupture joints such as tear-open lines (7 and/or 12, 13) in its walls (5 and 6) adjoining and/or opposing said condom open end, and wherein said ends (3a) protruding beyond the rolled-up condom (2) of the strip-like members (3) uniformly distributed over the condom's periphery and rolled into the condom (2) by their opposite ends are fixed around said condom substantially coaxial therewith inside the outer wrap, characterized by the fact that the ends (3a) not so rolled into the condom are joined to each other and formed into the material of said outer wrap 4), that at least one rupture joint (9) such as a tear-open line extending transversely to the rolled-up condom is provided in the area of said coherent ends (3a) of said strip-like members (3) to have the outer wrap (4) torn open by said members (3), or that the ends not rolled into the condom of said strip-like members (3) are attached to a ring (10) of soft, flexible material with transverse gap (11) for tearing off said outer wrap (4) which ring is integrated into the material of the outer wrap (4) and disposed substantially coaxial with said rolled-up condom (2).

2. Package unit according to claim 1, characterized by the fact that said strip-like members (3) are arranged and formed such as to laterally overlap each other in an upper portion thereof.

3. Package unit according to claim 1, characterized by the fact that said outer wrap (4) has a rectangular, square, circular or oval contour and that said rupture joints such as tear-open lines (7) in the walls (5, 6) of the outer wrap (4) are provided in the form of a diagonally extending cross.

4. Package unit according to claim 1, characterized by the fact that said rupture joints such as tear-open lines in the walls (5, 6) of the outer wrap (4) are of circular configuration.

5. Package unit according to claim 1, characterized by the fact that said rupture joints (7) in the walls (5, 6) of the outer wrap (4) are arranged to extend along the centerlines (12 and/or 13) thereof and that tear-open aids (14) in the form of notches, recesses or such like are provided in the areas of transition thereof into the marginal edges of the outer wrap (4).

6. Package unit according to claim 1, characterized by the fact that said strip-like members (3) are tapering towards their free ends (3b).

7. Package unit according to claim 1, characterized by the fact that the free ends (3b) of said strip-like members are rounded off.

8. Package unit according to claim 1, characterized by the fact that said strip-like members (3) are in the form of large-width, thin plastic tapes.

9. Package unit according to claim 1, characterized by the fact that the type of plastic material used is polypropylene or polyethylene.

10. Package unit according to claim 1, characterized by the fact that an area weakened by rupture joints with tear-off tab is provided in the lower wall (6) of the outer wrap (4).

## Revendications

1. Unité d'emballage composée d'un emballage extérieur (4) et, disposé à l'intérieur, d'un condom (2) ayant la forme d'une gaine hautement élastique, tubulaire, fermée à une extrémité qui, en position emballée, est enroulée autour d'un bourrelet annulaire situé à l'extrémité ouverte de la gaine et avec laquelle les éléments en forme de languette (3) en matériau mince, flexible et souple facilitant le déroulement sont enroulés sur la plus grande partie de leur longueur, l'emballage extérieur (4) étant composé d'un matériau déchirable et présentant dans les parois de l'ouverture ou faisant face à l'ouverture du condom (5 et 6) des points destinés à la rupture, p.ex. des lignes destinées à la rupture (7 ou 12, 13), les extrémités (3 a) débordant le condom enroulé (2) des éléments (3) en forme de languette disposés de manière régulière sur l'ensemble du condom et dont les autres extrémités ouvertes (3 b) sont enroulées dans le condom (2) étant pour l'essentiel fixées autour de l'axe du condom (2) à l'intérieur de l'emballage extérieur (4),
caractérisée par le fait
que les extrémités non enroulées (3 a) des éléments en forme de languette (3) sont cohérentes et prises dans le matériau de l'emballage extérieur (4) et en outre par le fait qu'au moins un point destiné à la rupture, p.ex. une ligne destinée à la rupture (9) disposée de manière transversale par rapport au condom enroulé est prévue dans le secteur des extrémités cohérentes (3 a) des éléments en forme de languette (3) pour ouvrir l'emballage extérieur (4) avec les éléments en forme de languette, ou que les extrémités non enroulées des éléments en forme de languette (3) sont fixées à un anneau (10) en matériau souple et flexible présentant une interruption (11) transversale destinée à ouvrir l'emballage extérieur (4), l'anneau étant fixé dans le matériau de l'emballage extérieur (4) et étant disposé pour l'essentiel autour de l'axe du condom enroulé (2).

2. Unité d'emballage d'après la revendication 1,
caractérisée par le fait
que les éléments en forme de languette (3) sont disposés et façonnés de sorte qu'ils se chevauchent latéralement dans la zone supérieure.

3. Unité d'emballage d'après la revendication 1,
caractérisée par le fait
que l'emballage extérieur (4) présente des contours rectangulaires ou carrés ou ronds ou ovales et que les points destinés à la rupture, p.ex. les lignes destinées à la rupture (7), sont disposées dans les parois (5, 6) de l'emballage extérieur (4) en forme de croix diagonale.

4. Unité d'emballage d'après la revendication 1,
caractérisée par le fait
que les points destinés à la rupture, p.ex. les lignes destinées à la rupture, sont disposées en cercle dans les parois (5, 6) de l'emballage extérieur (4).

5. Unité d'emballage d'après la revendication 1,
caractérisée par le fait
que les points destinés à la rupture (7) dans les parois (5, 6) de l'emballage extérieur (4) courent le long des lignes médianes (12 et/ou 13) de celui-ci et que, dans la zone ou celles-ci débouchent, sont disposés dans les bords latéraux de l'emballage extérieur (4) des éléments destinés à faciliter la déchirure (14) sous forme d'entailles, d'incisions, d'évidements ou autres.

6. Unité d'emballage d'après la revendication 1,
caractérisée par le fait
que les éléments en forme de languette (3) ont des extrémités libres (3 b) allant en se rétrécissant.

7. Unité d'emballage d'après la revendication 1,
caractérisée par le fait
que les éléments en forme de languette (3) ont des extrémités libres (3 b) arrondies.

8. Unité d'emballage d'après la revendication 1,
caractérisée par le fait
que les éléments en forme de languette (3) sont des rubans larges et minces en matière plastique.

9. Unité d'emballage d'après la revendication 1,
caractérisée par le fait
que la matière plastique utilisée est du polypropylène ou du polyéthylène.

10. Unité d'emballage d'après la revendication 1,
caractérisée par le fait
qu'une zone marquée par des points destinés à la rupture est disposée sur la paroi inférieure (6) de l'emballage extérieur (4) avec une languette permettant de l'arracher.
